# EUROPÄISCHE PATENTSCHRIFT

(11) **EP 4 612 121 B1**
(45) Veröffentlichungstag und Bekanntmachung des Hinweises auf die Patenterteilung: **12.08.2026**
(21) Anmeldenummer: 23800344.6
(22) Anmeldetag: 27.10.2023
(51) Int. Cl.: C07C 209/26, C07C 211/50, C08K 5/18

(54) **VERFAHREN ZUR HERSTELLUNG VON N,N'-DIALKYL-P-PHENYLENDIAMIN**
METHOD FOR PRODUCING N,N'-DIALKYL-P-PHENYLENEDIAMINE
PROCÉDÉ DE PRÉPARATION DE N,N'-DIALKYL-P-PHÉNYLÈNEDIAMINE

(30) Priorität: 02.11.2022 EP 22205113
(43) Veröffentlichungstag der Anmeldung: 10.09.2025
(73) Patentinhaber: LANXESS Deutschland GmbH, 50569 Köln (DE)
(72) Erfinder: WIEDEMEIER-JARAD, Melanie, 41540 Dormagen (DE); GRAF, Holger, 42781 Haan (DE); STUCKE, Nadja, 25462 Rellingen (DE); BÜHRLE, Miriam, 68723 Schwetzingen (DE); MINDACH, Olaf, 51379 Leverkusen (DE)
(86) Internationale Anmeldenummer: PCT/EP2023/080114
(87) Internationale Veröffentlichungsnummer: WO 2024/094570

(56) Entgegenhaltungen:
- WO-A1-2022/207729
- FR-A- 1 324 155
- US-A- 2 835 705
- US-A- 3 304 285
- US-A- 4 140 718

## Beschreibung

Die vorliegende Erfindung betrifft ein neues Verfahren zur Herstellung von *N,N'*-Dialkyl-*p-*phenylendiamin der Formel (I). Eine weitere Offenbarung ist die Verwendung des *N,N'*-Dicyclohexyl-*p-*phenylendiamins in einer Reinheit von größer 95,0 Gew.-% als Alterungsschutzmittel in Kautschukmischungen, -vulkanisaten und daraus erhältlichen Formkörpern, insbesondere Reifen.

*N,N'*-Dicyclohexyl-p-phenylendiamin ist hervorragend geeignet, Vulkanisate auf Basis von Naturkautschuken oder Synthesekautschuken gegen Alterungsprozesse, bedingt durch die Einwirkung von Sauerstoff und Ozon, zu schützen. Es migriert an die Oberfläche des Vulkanisats und schützt dort den Kautschuk vor den zuvor genannten Einwirkungen.

CN1370768A beschreibt ein Verfahren zur Herstellung von N,N'-Di-sec-alkyl-p-phenylendiamin. p-Nitroanilin oder p-Phenylendiamin werden mit aliphatischen C₃-C₁₂ Ketonen in Gegenwart von CuO, Cr₂O₃ und BaO umgesetzt.

CN1947837 A beschreibt ein Verfahren zur Herstellung von *N,N'*-Dialkyl-*p*-phenylendiamin. Als Katalysator wird ein Gemisch aus drei Komponenten gewählt: M1 (Ni, Co oder Fe), M2 (Re, Pd und/oder Ru) sowie M3 (Li, Ma, K, Rb, Ca, Mg, Sr oder Ba).

Nachteilig an beiden zuvor genannten Verfahren ist, dass mehrere Katalysatoren eingesetzt werden, die teils teuer und umweltbedenklich sind.

In CN105061214A wird ein Verfahren zur Herstellung von N,N'-Di-sec-butyl-p-phenylendiamin beschrieben, ausgehend von einer reduktiven Alkylierung von p-Nitroanilin und eines Ketons in Anwesenheit eines Platin-Katalysators sowie Aktivkohle. In diesem Verfahren wird nach Zugabe von p-Nitroanilin und Keton, hier Butanon, Aktivkohle zugegeben, um mögliche Verunreinigungen herauszufiltern. Erst nach Abtrennung der Aktivkohle durch Filtration wird der Katalysator zugegeben. Es wird eine Umsatzrate von 98.7% beschrieben, jedoch ist nicht beschrieben, in welcher Reinheit das Produkt letztlich vorliegt. Auch ist in dem beschriebenen Verfahren der Einsatz von Aktivkohle zur Reinigung der Ausgangsstoffe erforderlich. Ein Einsatz von Aktivkohle birgt jedoch einige Nachteile: Die Aktivkohle muss in einem weiteren Reaktionsschritt aus dem Reaktionsgemisch entfernt werden, was einen Mehraufwand sowie zusätzliche Reaktionsdauer erfordert. Darüber hinaus birgt Aktivkohle die Gefahr, Verunreinigungen im Reaktionsgemisch zu hinterlassen sowie beispielsweise Rohrleitungen und Filter zu verstopfen. Auch die Handhabung von Aktivkohlepulver ist problematisch, da der entstehende Staub einen erhöhten Aufwand aufgrund erhöhter Sicherheitsmaßnahmen erfordert.

In US4140718A wird ein Verfahren zur Herstellung von *N,N'*-Dialkyl-*p*-phenylendiaminen wie auch *N,N'*-Dicyclohexyl-p-phenylendiamin beschrieben, ausgehend von einer Umsetzung von p-Nitroanilin und eines Ketons in Anwesenheit von Wasserstoff und einem Hydrierkatalysator. Darin wird beschrieben, dass sich p-Nitroanilin schlecht in organischen Lösungsmitteln löst und es bei Verwendung von Ketonen mit geringem Molekulargewicht, wozu auch Cyclohexanon zählt, als Alkylierungsmittel bei Verwendung im Überschuss zum Ersatz beider Amin-Wasserstoffatome des aromatische Amins kommt. Es werden verschiedene Ether als Lösungsmittel eingesetzt. Die Reinheiten der Produkte sind mit 93 - 97% beschrieben. Allerdings werden die mono- und trialkylierten Nebenprodukte in Summe in 3 - 7 % erhalten, einzeln in jeweils mindestens 1%. Diese Nebenprodukte sind sehr unerwünscht, da sie die Migration des *N,N'*-Dialkyl-*p*-phenylendiamins, bevorzugt des *N,N'*-Dicyclohexyl-*p*-phenylendiamins, an die Oberfläche des Vulkanisats stören und damit im Fall von *N,N'*-Dicyclohexyl-p-phenylendiamin seine Wirksamkeit einschränken. So bildet das monoalkylierte Nebenprodukt aufgrund seiner freien Amin-Gruppe Wasserstoffbrückenbindungen mit anderen Molekülen, und das trialkylierte Nebenprodukt ist sterisch sehr anspruchsvoll und sperrig - beides Faktoren, die die oben beschriebene Migration behindern. Darüber hinaus wird in diesem Verfahren als Katalysator Platin aufgezogen auf Aluminium eingesetzt. Nachteilig daran ist, dass die Herstellung von Aluminium oft teuer und umweltschädlich ist. Auch ist die Hydrierzeit mit 4 - 5,2 Stunden relativ lang.

Es bestand daher Bedarf an einem Verfahren zur Herstellung von *N,N'*-Dialkyl-*p-*phenylendiamin, bevorzugt von *N,N'*-Dicycloalkyl-*p*-phenylendiamin, besonders bevorzugt von *N,N'*-Dicyclohexyl-*p*-phenylendiamin, das die vorgenannten Nachteile des Standes der Technik überwindet.

Aufgabe der vorliegenden Erfindung war es, ein Verfahren zur Herstellung von N,N'-Dialkyl-p-phenylendiamin, bevorzugt von *N,N'*-Dicycloalkyl-*p*-phenylendiamin, besonders bevorzugt von *N,N'*-Dicyclohexyl-*p*-phenylendiamin, bereit zu stellen, das die Nachteile des Standes der Technik überwindet, und *N,N'*-Dialkyl-*p*-phenylendiamin, bevorzugt N,N'-Dicycloalkyl-p-phenylendiamin, besonders bevorzugt *N,N'*-Dicyclohexyl-*p*-phenylendiamin, in hoher Reinheit und mit nur geringen Mengen der mono- und trialkylierten Nebenprodukte (im Rahmen dieser Erfindung bevorzugt *N*-Alkyl-*p*-phenylendiamin sowie *N,N,N'*-Trialkyl-*p-*phenylendiamin), herstellbar macht.

Die Reinheit der erhaltenen Produkte wird im Rahmen der vorliegenden Erfindung bevorzugt mittels Gaschromatographie gemäß ASTM-D 4937 ermittelt. Sie entspricht einer Reinheit in Gew.-%.

Eine hohe Reinheit im Rahmen der vorliegenden Erfindung liegt vor, wenn im Fall der Ermittlung der Reinheit über die Gaschromatographie das nach dem erfindungsgemäßen Verfahren hergestellte Produkt eine Reinheit von größer 95,0 Gew.-%, bevorzugt größer/gleich 96,0 Gew.-%, meist bevorzugt größer als 97,0 Gew.-%, aufweist.

Eine geringe Menge der mono- und trialkylierten Nebenprodukte (im Rahmen dieser Erfindung bevorzugt *N*-Alkyl-*p*-phenylendiamin sowie *N,N,N'*-Trialkyl-*p*-phenylendiamin) beträgt im Rahmen der vorliegenden Erfindung in Summe beider Nebenprodukte kleiner als 3,0 Gew.-%, bevorzugt kleiner als 2,5 Gew.-%, besonders bevorzugt kleiner als 1,5 Gew.-%, meist bevorzugt kleiner als 1,0 Gew.-%. Eine geringe Menge *N*-Alkyl-*p-*phenylendiamin liegt vor, wenn dies zu weniger als 1,0 Gew.-%, bevorzugt weniger als 0,5 Gew.-%, besonders bevorzugt weniger als 0,3 Gew.-% im Produkt enthalten ist. Eine geringe Menge *N,N,N'*-Trialkyl-*p*-phenylendiamin liegt vor, wenn dies zu weniger als 3 Gew.-%, bevorzugt weniger als 1,5 Gew.-%, besonders bevorzugt weniger als 1,0 Gew.-% im Produkt enthalten ist.

Überraschenderweise wurde gefunden, dass die Umsetzung einer Verbindung der Formel (II) mit wenigstens einem Keton der Formel (III) und einem Lösungsmittel der Formel (IVb) oder (IVc), oder Mischungen davon, in Gegenwart eines Hydrierkatalysators und Wasserstoff zu hohen Reinheiten der entsprechenden *N,N'*-Dialkyl-*p*-phenylendiamine der Formel (I) führt, bei Erhalt nur geringer Mengen an mono- und trialkylierten Nebenprodukten.

### Verfahren

Gegenstand der vorliegenden Erfindung ist daher ein Verfahren zur Herstellung von *N,N'-*Dialkyl-p-phenylendiamin der Formel (I) 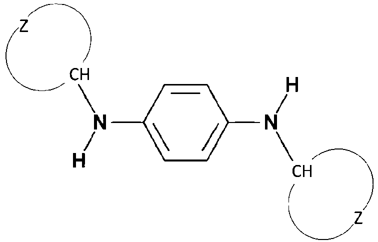 wobei eine Verbindung der Formel (II) 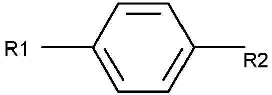 wobei R1 und R2 jeweils gleich oder verschieden sind und ausgewählt sind aus der Gruppe bestehend aus -NH₂ und -NO₂,
und wenigstens ein Keton der Formel (III) 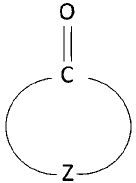
in Gegenwart eines Hydrierkatalysators und Wasserstoff umgesetzt werden,
wobei Z in den Formeln (I) und (III) gleich ist und
   a) für eine Alkylenkette, bevorzugt eine lineare Alkylenkette, steht, die mit dem an Z angrenzenden Kohlenstoffatom einen cyclischen Ring bildet, und wobei die Alkylenkette von Z bevorzugt C₄-C₆-Alkylen, besonders bevorzugt C₅-Alkylen, ist,
      oder
   b) für zwei Alkylreste, bevorzugt lineare Alkylreste, steht, welche jeweils mit dem an Z angrenzenden Kohlenstoffatom verbunden sind und gemeinsam mit diesem Kohlenstoffatom eine Alkylkette, bevorzugt eine lineare Alkylkette, bilden, und wobei die zwei Alkylreste von Z gemeinsam die Summenformel CₙH₂ₙ₊₂ bilden, bevorzugt mit n=4-6, besonders bevorzugt mit n=5,
dadurch gekennzeichnet, dass die Umsetzung in Gegenwart eines Lösungsmittels der Formel (IVb) 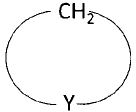
oder der Formel (IVc) 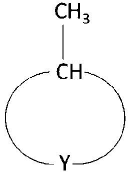
oder Mischungen davon,
erfolgt,
wobei Y in den Formeln (IVb) und (IVc) gleich oder verschieden, bevorzugt gleich, ist und
   a) für eine Alkylenkette, bevorzugt eine lineare Alkylenkette, steht, die mit dem an Y angrenzenden Kohlenstoffatom einen cyclischen Ring bildet, und wobei die Alkylenkette von Y bevorzugt C₄-C₆-Alkylen, besonders bevorzugt C₅-Alkylen ist,
      oder
   b) für zwei Alkylreste, bevorzugt lineare Alkylreste, oder einen Alkylrest und ein Wasserstoffatom steht, welche jeweils mit dem an Y angrenzenden Kohlenstoffatom verbunden sind und gemeinsam mit diesem Kohlenstoffatom eine Alkylkette, bevorzugt eine lineare Alkylkette, bilden, und wobei die zwei Alkylreste oder der eine Alkylrest und das Wasserstoffatom gemeinsam die Summenformel CₙH₂ₙ₊₂ bilden, bevorzugt mit n=4-6, besonders bevorzugt mit n=5.

Der Begriff "Alkylen" steht in obigen Definitionen von Y und Z bevorzugt für CₙH₂ₙ.

In dem erfindungsgemäßen Verfahren können Y in den Formeln (IVb) und (IVc) und Z in den Formeln (I) und (III) gleich oder verschieden sein. Bevorzugt sind Y und Z in den Formeln (I), (III), (IVb) und (IVc) gleich.

In einer bevorzugten Ausführungsform des erfindungsgemäßen Verfahrens sind Z und Y in den Formeln (I) und (III) gleich.

In einer weiteren bevorzugten Ausführungsform des erfindungsgemäßen Verfahrens sind Z und Y in den Formeln (I), (III) und (IVb) gleich.

In einer weiteren bevorzugten Ausführungsform des erfindungsgemäßen Verfahrens sind Z und Y in den Formeln (I), (III) und (IVc) gleich.

In einer weiteren Ausführungsform der vorliegenden Erfindung ist Z in den Formeln (I) und (III) gleich und verschieden von Y in den Formeln (IVb) bzw. (IVc).

Das erfindungsgemäße Verfahren ist ein Verfahren zur Herstellung von *N,N'*-Dialkyl-*p-*phenylendiamin der Formel (I).

Z kann in Formel (I) für zwei Alkylreste, bevorzugt lineare Alkylreste, stehen, welche jeweils mit dem an Z angrenzenden Kohlenstoffatom verbunden sind und gemeinsam mit diesem Kohlenstoffatom eine Alkylkette, bevorzugt eine lineare Alkylkette, bilden, und wobei die zwei Alkylreste von Z gemeinsam die Summenformel CₙH₂ₙ₊₂ bilden, bevorzugt mit n=4-6, besonders bevorzugt mit n=5.

Demnach kann das erfindungsgemäße Verfahren ein Verfahren zur Herstellung von N,N'-Alkyl-p-phenylendiamin sein, bevorzugt von N,N'-Pentyl-, N,N'-Hexyl-, N,N'- Heptyl-p-phenylendiamin, besonders bevorzugt von *N,N'*-Hexyl-*p*-phenylendiamin.

In einer bevorzugten Ausführungsform des erfindungsgemäßen Verfahrens steht Z in Formel (I) für eine Alkylenkette, bevorzugt eine lineare Alkylenkette, die mit dem an Z angrenzenden Kohlenstoffatom einen cyclischen Ring bildet, und wobei die Alkylenkette von Z bevorzugt C₄-C₆-Alkylen, besonders bevorzugt C₅-Alkylen ist.

Bevorzugt ist das erfindungsgemäße Verfahren demnach ein Verfahren zur Herstellung von *N,N'*-Cycloalkyl-*p*-phenylendiamin.

Besonders bevorzugt steht Z in Formel (I) für eine lineare Alkylenkette, die mit dem an Z angrenzenden Kohlenstoffatom einen cyclischen Ring bildet, und wobei die lineare Alkylenkette C₄-C₆-Alkylen, meist bevorzugt C₅-Alkylen ist.

Besonders bevorzugt ist das erfindungsgemäße Verfahren demnach ein Verfahren zur Herstellung von *N,N'*-Cyclopentyl-*p*-phenylendiamin, *N,N'*-Cyclohexyl-*p*-phenylendiamin und *N,N'*-Cycloheptyl-*p*-phenylendiamin, meist bevorzugt von *N,N'*-Cyclohexyl-*p-*phenylendiamin.

In dem erfindungsgemäßen Verfahren wird die Verbindung der Formel (II) eingesetzt, wobei R1 und R2 jeweils gleich oder verschieden sind und ausgewählt sind aus der Gruppe bestehend aus -NH₂ und -NO₂.

Bevorzugt sind R1 und R2 gleich und -NH₂, oder verschieden und -NH₂ und -NO₂, besonders bevorzugt verschieden und -NH₂ und -NO₂. Besonders bevorzugt ist die Verbindung der Formel (II) demnach para-Nitroanilin.

Es können beliebige Ketone der Formel (III) eingesetzt werden.

Y kann in dem wenigstens einen Keton der Formel (III) für zwei Alkylreste, bevorzugt lineare Alkylreste, stehen, welche jeweils mit dem an Z angrenzenden Kohlenstoffatom verbunden sind und gemeinsam mit diesem Kohlenstoffatom eine Alkylkette, bevorzugt eine lineare Alkylkette, bilden, und wobei die zwei Alkylreste von Z gemeinsam die Summenformel CₙH₂ₙ₊₂ bilden, bevorzugt mit n=4-6, besonders bevorzugt mit n=5.

Demnach kann als wenigstens ein Keton der Formel (III) ein Keton ausgewählt aus der Gruppe Pentanon, Hexanon und Heptanon eingesetzt werden, bevorzugt ausgewählt aus Pentan-2-on, Pentan-3-on, Hexan-2-on, Hexan-3-on, Heptan-2-on, Heptan-3-on und Heptan-4-on.

Gemäß obiger Bevorzugung von Y in Formel (III) ist das in Schritt a) eingesetzte wenigstens eine Keton der Formel (III) bevorzugt ein cyclisches Keton, besonders bevorzugt ausgewählt aus Cyclopentanon, Cyclohexanon und Cycloheptanon.

Meist bevorzugt ist das Keton der Formel (III) demnach Cyclohexanon.

Bevorzugt wird in dem erfindungsgemäßen Verfahrens ein Keton der Formel (III) eingesetzt.

Es können beliebige Lösungsmittel der Formeln (IVb) oder (IVc) oder Mischungen davon eingesetzt werden.

Y kann in dem Lösungsmittel der Formel (IVb) oder (IVc) für zwei Alkylreste, bevorzugt lineare Alkylreste, oder einen Alkylrest und ein Wasserstoffatom stehen, welche jeweils mit dem an Y angrenzenden Kohlenstoffatom verbunden sind und gemeinsam mit diesem Kohlenstoffatom eine Alkylkette, bevorzugt eine lineare Alkylkette, bilden, und wobei die zwei Alkylreste oder der eine Alkylrest und das Wasserstoffatom gemeinsam die Summenformel CₙH₂ₙ₊₂ bilden, bevorzugt mit n=4-6, besonders bevorzugt mit n=5.

Bevorzugt steht Y in den Formeln (IVb) oder (IVc) für eine Alkylenkette, bevorzugt eine lineare Alkylenkette, die mit dem an Y angrenzenden Kohlenstoffatom einen cyclischen Ring bildet, und wobei die Alkylenkette von Y bevorzugt C₄-C₆-Alkylen, besonders bevorzugt C₅-Alkylen ist.

Gemäß obiger Bevorzugung von Y in Formel (IVb) ist das eingesetzte Lösungsmittel der Formel (IVb) bevorzugt ausgewählt aus Cyclopentan, Cyclohexan und Cycloheptan, oder Mischungen davon.

Meist bevorzugt ist das Lösungsmittel der Formel (IVb) Cyclohexan.

Gemäß obiger Bevorzugung von Y in Formel (IVc) ist das eingesetzte Lösungsmittel der Formel (IVc) bevorzugt ausgewählt aus Methylcyclopentan, Methylcyclohexan und Methylcycloheptan, oder Mischungen davon.

Meist bevorzugt ist das Lösungsmittel der Formel (IVc) Methylcyclohexan.

Das Lösungsmittel der Formel (IV) ist ein Lösungsmittel der Formel (IVb) oder (IVc), oder Mischungen davon, besonders bevorzugt ausgewählt aus Cyclohexan und Methylcyclohexan.

Meist bevorzugt ist das Lösungsmittel der Formel (IV) Methylcyclohexan.

Ether, bevorzugt ausgewählt aus der Gruppe bestehend aus Monoethern aus Gylkolen, Diethern aus Gylkolen und cyclischen Diethern, sind in dem erfindungsgemäßen Verfahren bevorzugt in einem molaren Verhältnis zu der Verbindung der Formel (II) von weniger als 0,2 : 1,0, besonders bevorzugt von weniger als 0,05 :1, ganz besonders bevorzugt von weniger als 0,01 : 1,0 zugegen. Meist bevorzugt sind in dem erfindungsgemäßen Verfahren keine Ether, bevorzugt ausgewählt aus der Gruppe bestehend aus Monoethern aus Gylkolen, Diethern aus Gylkolen und cyclischen Diethern, zugegen.

Die zuvor genannten Ausführungen und Bevorzugungen von Z in den Formeln (I) und (III) und Y in den Formeln (IVb) und (IVc) gelten folglich nicht nur für die Umsetzung in dem erfindungsgemäßen Verfahren, sondern auch für die sich optional anschließende Aufarbeitung.

Der in dem erfindungsgemäßen Verfahren eingesetzte Hydrierkatalysator kann ein für Hydrierungen aus dem Stand der Technik bekannter und gängiger Katalysator sein.

Bevorzugt enthält der Hydrierkatalysator ein Metall ausgewählt aus der Gruppe bestehend aus Platin, Palladium und Nickel, besonders bevorzugt Platin.

Der Hydrierkatalysator kann auf einem festen Trägermaterial aufgezogen sein, beispielsweise Kohlenstoff, Aluminium, Bariumsulfat oder Calciumcarbonat. Bevorzugt ist der Hydrierkatalysator ein Katalysator auf Kohlenstoff.

Besonders bevorzugt ist der Hydrierkatalysator Platin auf Kohlenstoff (Pt/C).

Der Hydrierkatalysator kann eine beliebige Menge Platin enthalten. Bevorzugt enthält der Hydrierkatalysator 1 - 10 Gew.-% Platin, besonders bevorzugt 1,5 - 5 Gew.-% Platin, ganz besonders bevorzugt 2 - 4 Gew.-% Platin, ganz besonders bevorzugt 2,5 - 3,5 Gew.-% Platin, meist bevorzugt 3 Gew.-% Platin.

Der Einsatz des Hydrierkatalysators Platin auf Kohlenstoff löst die weitere Aufgabe, ein umweltfreundlicheres Verfahren bereitzustellen, verglichen mit dem in US4140718A offenbarten, welches Platin auf Aluminium als Hydrierkatalysator einsetzt.

Der Hydrierkatalysator kann Wasser enthalten oder nicht enthalten. Bevorzugt enthält der Katalysator Wasser, besonders bevorzugt 30 - 70 Gew.-%, ganz besonders bevorzugt 50 - 70 Gew.-%, meist bevorzugt 60 - 65 Gew.-%.

Der Hydrierkatalysator kann vor dem Einsatz in dem erfindungsgemäßen Verfahren entwässert werden. Die Entwässerung kann auf verschiedene bekannte Weisen erfolgen, beispielsweise über eine azeotrope Destillation. Bevorzugt erfolgt die Entwässerung über eine azeotrope Destillation. Dafür können verschiedene Lösungsmittel und Lösungsmittelgemische eingesetzt werden.

Die zur Umsetzung in dem erfindungsgemäßen Verfahren eingesetzten Komponenten können in verschiedenen Verhältnissen zueinander eingesetzt werden.

Bevorzugt wird das Keton der Formel (III) im molaren Verhältnis 1,2 : 1,0 bis 5,0 :1,0, besonders bevorzugt 1,5 : 1,0 bis 3,0 : 1,0, ganz besonders bevorzugt 1,8 : 1,0 bis 2,5 : 1,0, zu der Verbindung der Formel (II) eingesetzt.

Bevorzugt wird das Lösungsmittel der Komponente (IV) im molaren Verhältnis 1,5 : 1,0 bis 10,0 :1,0, besonders bevorzugt 2,0 : 1,0 bis 8,0 : 1,0, ganz besonders bevorzugt 3,0 : 1,0 bis 5,0 : 1,0, zu der Verbindung der Formel (II) eingesetzt.

Bevorzugt wird der Hydrierkatalysator im Gewichtsverhältnis 1,0 : 100,0 bis 6,0 :100,0, besonders bevorzugt 2,0 : 100,0 bis 6,0 : 100,0, ganz besonders bevorzugt 2,0 : 1,0 bis 4,0 : 100,0, zu der Verbindung der Formel (II) eingesetzt.

Die für die Umsetzung eingesetzten Komponenten können ohne eine vorherige Reinigung eingesetzt werden oder vor der Umsetzung gereinigt werden. Bevorzugt werden die für die Umsetzung eingesetzten Komponenten ohne eine vorherige Reinigung eingesetzt. Besonders bevorzugt werden die für die Umsetzung eingesetzten Komponenten in Reinheiten von ≥ 90%, ganz besonders bevorzugt ≥ 95%, ganz besonders bevorzugt ≥ 98%, meist bevorzugt ≥ 99% eingesetzt.

Die für die Umsetzung eingesetzten Komponenten können in beliebiger Reihenfolge vorgelegt werden.

Bevorzugt werden zunächst die Verbindungen der Formeln (II), (III) und (IV) vorgelegt.

Bevorzugt werden die eingesetzten Komponenten des erfindungsgemäßen Verfahrens vor der Umsetzung einer Schutzgasatmosphäre ausgesetzt, wie beispielsweise einer Argon- oder Stickstoffatmosphäre, bevorzugt einer Stickstoffatmosphäre.

Die Umsetzung erfolgt bevorzugt bei einer Temperatur von 100 - 170 °C, besonders bevorzugt bei 90 - 150 °C, ganz besonders bevorzugt bei 95 - 140 °C, meist bevorzugt bei 125 - 135 °C.

Die Umsetzung erfolgt bevorzugt bei einem Wasserstoffdruck von 5 - 30 bar, besonders bevorzugt von 10 - 20 bar, ganz besonders bevorzugt von 13 - 17bar.

Die Umsetzung erfolgt bevorzugt solange, bis keine weitere Aufnahme von Wasserstoff mehr detektiert werden konnte. Die Detektion der Wasserstoffaufnahme erfolgt bevorzugt durch Kontrolle der Druckänderung an einem Manometer. Besonders bevorzugt erfolgt die Detektion in Abständen von weniger als 60 min, ganz besonders bevorzugt von weniger als 45 min, meist bevorzugt von weniger als 35 min.

Die Umsetzungsdauer beträgt bevorzugt 50 min bis 400 min, besonders bevorzugt 80 min bis 300 min, ganz besonders bevorzugt 100 bis 270 min, meist bevorzugt 100 bis 200 min.

Nach der erfolgten Umsetzung kann das Reaktionsgemisch noch weiter gerührt werden. Bevorzugt erfolgt ein solches Nachrühren für 10 min bis 200 min, besonders bevorzugt für 15 min bis 100 min, ganz besonders bevorzugt für 25 min bis 70 min.

In einer bevorzugten Ausführungsform des erfindungsgemäßen Verfahrens ist das Verfahren ein Verfahren zur Herstellung von *N,N'*-Dicycloalkyl-*p*-phenylendiamin der Formel (I), wobei Y und Z in den Formeln (I), (III), (IVb) und (IVc) gleich sind und für eine lineare Alkylenkette stehen, welche bevorzugt C₅-Alkylen ist.

Besonders bevorzugt ist das Lösungsmittel der Formel (IV) in der zuletzt genannten bevorzugten Ausführungsform ein Lösungsmittel der Formel (IVb).

Diese besonders bevorzugte Ausführungsform löst die weitere Aufgabe, ein effizienteres Verfahren bereitzustellen, verglichen mit dem in US4140718A offenbarten, welches Hydrierzeiten von 4 - 5,2 Stunden benötigt.

Bevorzugt ist das erfindungsgemäße Verfahren ein Verfahren zur Herstellung von N,N'-Dialkyl-p-phenylendiamin der Formel (I) in einer Reinheit von größer 95,0 Gew.-%, besonders bevorzugt größer/gleich 96,0 Gew.-%, meist bevorzugt größer als 97,0 Gew.-%, bevorzugt ermittelt mittels Gaschromatographie gemäß ASTM-D 4937.

Im Rahmen dieser Anmeldung ist der Ausdruck "Herstellung von *N,N'*-Dialkyl-*p-*phenylendiamin der Formel (I) in einer Reinheit von größer 95,0 Gew.-%," gleichbedeutend mit "Herstellung einer Zusammensetzung enthaltend *N,N'*-Dialkyl-*p*-phenylendiamin der Formel (I) zu größer 95,0 Gew.-%". Entsprechendes gilt für analoge Textstellen in dieser Anmeldung wie auch im Abschnitt "Verwendung".

Bevorzugt ist das erfindungsgemäße Verfahren ein Verfahren zur Herstellung von N,N'-Dialkyl-p-phenylendiamin der Formel (I), welches die Verbindungen *N*-Alkyl-*p-*phenylendiamin sowie *N,N,N'*-Trialkyl-*p*-phenylendiamin in Summe kleiner als 3,0 Gew.-%, bevorzugt kleiner als 2,5 Gew.-%, besonders bevorzugt kleiner als 1,5 Gew.-%, meist bevorzugt kleiner als 1,0 Gew.-%, aufweist, bevorzugt ermittelt mittels Gaschromatographie gemäß ASTM-D 4937.

### Aufarbeitung

Nach der Umsetzung kann die erhaltene Reaktionsmischung auf verschiedene Weisen aufgearbeitet werden, die dem Fachmann bekannt sind.

In einer bevorzugten Ausführungsform des erfindungsgemäßen Verfahrens schließt sich der Umsetzung die Entfernung des Hydrierkatalysators aus der Reaktionsmischung erhalten nach der Umsetzung an.

Die Entfernung kann auf verschiedene, dem Fachmann bekannte Weisen erfolgen, wie beispielsweise Filtration, bevorzugt über eine Fritte oder Drucknutsche, oder Absaugen. Bevorzugt erfolgt die Entfernung durch Filtration der Reaktionsmischung erhalten nach der Umsetzung, besonders bevorzugt durch Filtration der heißen Reaktionsmischung.

Nach Entfernung des Hydrierkatalysators aus der Reaktionsmischung kann das Lösungsmittel der Reaktionsmischung erhalten nach der Entfernung des Hydrierkatalysators auf verschiedene, dem Fachmann bekannte Weisen entfernt werden.

Bevorzugt erfolgt die Entfernung durch Destillation der Reaktionsmischung erhalten nach Entfernung des Hydrierkatalysators. Die Temperatur der Destillation ist abhängig von dem Siedepunkt des eingesetzten Lösungsmittels. Bevorzugt erfolgt die Destillation bei vermindertem Druck.

Das nach der Entfernung des Lösungsmittels wiedergewonnene Lösungsmittelgemisch kann für eine erneute Hydrierung eingesetzt werden.

In einer besonders bevorzugten Ausführungsform schließt sich der Umsetzung des erfindungsgemäßen Verfahrens die Entfernung des Hydrierkatalysators aus der Reaktionsmischung erhalten nach der Umsetzung an sowie die Entfernung des Lösungsmittels aus der Reaktionsmischung erhalten nach Entfernung des Hydrierkatalysators. Die Bestimmung der Reinheit des *N,N'*-Dialkyl-*p*-phenylendiamins der Formel (I) sowie die Bestimmung der Anteile an *N*-Alkyl-*p*-phenylendiamin sowie *N,N,N'-*Trialkyl-p-phenylendiamin, bevorzugt ermittelt mittels Gaschromatographie gemäß ASTM-D 4937, erfolgt bevorzugt nach Entfernung des Hydrierkatalysators und des Lösungsmittels.

Nach Entfernung des Lösungsmittels aus der Reaktionsmischung kann der erhaltene Destillationsrückstand auf verschiedene, dem Fachmann bekannte Weisen aufgearbeitet werden.

Bevorzugt wird der erhaltene Destillationsrückstand abgekühlt, wobei das *N,N'*-Dialkyl-*p-*phenylendiamins der Formel (I) auskristallisiert.

Besonders bevorzugt wird der erhaltene heiße Destillationsrückstand auf ein Kristallisierblech gegeben.

Optional kann das erhaltene *N,N'*-Dialkyl-*p*-phenylendiamin der Formel (I) im Anschluss getrocknet werden.

Die Trocknung kann auf verschiedene, dem Fachmann bekannte Weisen erfolgen. Bevorzugt erfolgt die Trocknung im Vakuum, besonders bevorzugt bei einem Druck von kleiner als 10 kPa, ganz besonders bevorzugt bei einem Druck kleiner als 5 kPa.

### Verwendung

Eine weitere Offenbarung ist die Verwendung von *N,N'*-Dicyclohexyl-p-phenylendiamin in einer Reinheit von größer 95,0 Gew.-%, bevorzugt größer/gleich 96,0 Gew.-%, meist bevorzugt größer als 97,0 Gew.-%, als Alterungsschutzmittel in Kautschukmischungen, - vulkanisaten und daraus erhältlichen Formkörpern, insbesondere Reifen, wobei die Reinheit bevorzugt ermittelt ist mittels Gaschromatographie gemäß ASTM-D 4937.

In einer weiteren Offenbarung weist das *N,N'*-Dicyclohexyl-p-phenylendiamin die Verbindungen *N*-Cyclohexyl-*p*-phenylendiamin und *N,N,N'*-Tricyclohexyl-*p-*phenylendiamin in Summe kleiner als 3,0 Gew.-%, bevorzugt kleiner als 2,5 Gew.-%, besonders bevorzugt kleiner als 1,5 Gew.-%, meist bevorzugt kleiner als 1,0 Gew.-%, auf.

In einer weiteren Offenbarung weist das *N,N'*-Dicyclohexyl-p-phenylendiamin die Verbindung *N*-Cyclohexyl-*p*-phenylendiamin in weniger als 0,5 Gew.-%, ganz besonders bevorzugt in weniger als 0,3 Gew.-% auf.

In einer weiteren Offenbarung weist das *N,N'*-Dicyclohexyl-p-phenylendiamin die Verbindung *N,N,N'*-Tricyclohexyl-*p*-phenylendiamin in weniger als 3 Gew.-%, ganz besonders bevorzugt in weniger als 1,5 Gew.-%, meist bevorzugt in weniger als 1,0 Gew.-% auf.

Die angeführten Vorzugsbereiche gelten für die Verwendung in Kautschukmischungen, - vulkanisaten und Formkörpern, insbesondere Reifen, analog.

Ebenso gelten die im Rahmen dieser Erfindung angeführten Beschreibungen und Vorzugsbereiche unabhängig davon, ob sie im Plural oder im Singular offenbart wurden.

Die Erfindung soll anhand der folgenden Beispiele erläutert werden, ohne diese jedoch darauf zu beschränken.

### Beispiele

### Verfahren zur Herstellung von N,N'-Dicyclohexyl-p-phenylendiamin

**Tabelle 1: Liste der Einsatzstoffe, Abkürzungen und Hersteller**

| **Handelsname** | **Erläuterung** | **Hersteller/ Vertrieb** |
|---|---|---|
| p-Nitroanilin | Rohstoff, Reinheit ≥ 99% | Sigma-Aldrich |
| Cyclohexanon | Rohstoff, Reinheit ≥ 99% | Sigma-Aldrich |
| Cyclohexanol | Lösemittel, Reinheit ≥ 99% | Sigma-Aldrich |
| Evonik Noblyst^{®} P2067 3% Pt/C | Hydrierkatalysator: Platin/Aktivkohle (3 Gew.-% Platin, 62,5% Gew.-% H₂O) | Evonik Industries |
| Cyclohexan | Lösemittel, Reinheit ≥ 99% | Sigma-Aldrich |
| Methylcyclohexan | Lösemittel, Reinheit ≥ 99% | Sigma-Aldrich |

**Tabelle 2: Einsatzmengen der Einsatzstoffe und Reaktionsparameter**

| | **Beispiel 1** | **Beispiel 2** | **Beispiel 3 (nicht erfindungsgemäß)** |
|---|---|---|---|
| p-Nitroanilin | 69 g (0,5 mol) | 69 g (0,5 mol) | 69 g (0,5 mol) |
| Cyclohexanon | 101 g (1,03 mol) | 101 g (1,03 mol) | 103 g (1,05 mol) |
| Katalysator | 2 g | 2 g | 4 g |
| Methylcyclohexan | 188 g (1,91 mol) | | |
| Cyclohexan | | 188 g (2,23 mol) | |
| Cyclohexanol | | | 188 g (1,88 mol) |
| Druck [bar H₂] | 15 | 15 | 15 |
| Temperatur [°C] | 130 | 130 | 100 |
| Aufnahme in bar H₂ | 135 | 130 | 146 |
| Umsetzungsdauer [min] | 120 | 152 | 262 |
| Nachrühren [min] | 60 | 60 | 30 |

### Herstellung von N,N'-Dicyclohexyl-p-phenylendiamin anhand der in Tabelle 2 angegebenen Einsatzmengen der Einsatzstoffe und Reaktionsparameter der Beispiele 1-3

Die folgende Durchführung erfolgte jeweils für die Beispiele 1-3.

### Umsetzung

Es wurden in einem 3 L-Alloy-Autoklaven (großer Blattrührer, 600 U/min) bei Raumtemperatur p-Nitroanilin, Cyclohexanon, der Katalysator und das jeweilige Lösungsmittel des jeweiligen Beispiels 1, 2 bzw. 3 vorgelegt.

Der Autoklav wurde verschlossen und mit Stickstoff gespült. Es wurde bei Raumtemperatur ein Wasserstoffdruck von 5 bar eingestellt und anschließend auf die jeweilige Temperatur der Beispiele 1, 2 bzw. 3 geheizt. Bei Erreichen der Temperatur wurde der Druck auf 15 bar H₂ gesteigert und es wurde bis zur Nullaufnahme hydriert (<1 bar H₂ Aufnahme in 30 min). Im Anschluss wurde noch 30 bzw. 60 min bei entsprechender Temperatur und Druck nachgerührt. Es wurde auf 75°C abkühlen gelassen und der Autoklave wurde entspannt und die Reaktionslösung entnommen.

### Aufarbeitung

Die Reaktionslösung erhalten nach der Umsetzung wurde heiß über eine Drucknutsche vom Katalysator filtriert.

Der wiedergewonnene Katalysator kann für eine erneute Hydrierung eingesetzt werden.

Die Reaktionslösung wurde bei 120°C/ 50 mbar von Leichtsiedern befreit. Das Destillat war zweiphasig. Die organische Phase bestand hauptsächlich aus dem Lösemittel (>99%) und kann für eine erneute Hydrierung eingesetzt werden.

Im Destillationssumpf erhielt man eine Schmelze, die heiß auf ein Kristallisierblech gegeben wurde. Nach Abkühlen wurde der auskristallisierte Feststoff abgeschlagen, zerkleinert und abgefüllt.

**Tabelle 3: Reinheiten*¹ der gemäß Beispiel 1, 2 und 3 hergestellten Produkte in [Gew.-%]**

| | **Beispiel 1** | **Beispiel 2** | **Beispiel 3 (nicht erfindungsgemäß)** |
|---|---|---|---|
| N-Cyclohexyl-p-phenylendiamin | 0,23 | 0,07 | 0,15 |
| N,N'-Dicyclohexyl-p-phenylendiamin | 96,45 | 97,73 | 96,00 |
| N,N,N'-Tricyclohexyl-p-phenylendiamin | 1,04 | 0,80 | 2,60 |

| | | | |
|---|---|---|---|
| *¹: **gemessen** mittels Gaschromatographie nach ASTM-D 4937 nach Entfernung des Hydrierkatalysators und des Lösungsmittels | | | |

### Ergebnisse

Wie aus Tabelle 3 ersichtlich ist, wurden *N,N'*-Dicyclohexyl-p-phenylendiamin in den Beispielen 1, 2 und 3 in einer hohen Reinheit von bis zu 97,73 Gew.-% hergestellt. Die unerwünschten Nebenprodukte *N*-Cyclohexyl-*p*-phenylendiamin sowie *N,N,N'-*Tricyclohexyl-p-phenylendiamin wurden nur in geringen Mengen erhalten, in Summe in jedem Beispiel unter 3 Gew.-%, in den Beispielen 1 und 2 sogar unter 1,5 Gew.-%, in Beispiel 2 sogar unter 1 Gew.-%. Das monoalkylierte Nebenprodukt *N*-Cyclohexyl-*p-*phenylendiamin ist sogar in allen Beispielen 1, 2 und 3 in weniger als 0,3 Gew.-% zugegen, das trialkylierte Nebenprodukt in Beispiel 2 sogar unter 1 Gew.-%. Beispiel 3 ist nicht erfindungsgemäß.

## Patentansprüche

1. Verfahren zur Herstellung von *N,N'*-Dialkyl-*p*-phenylendiamin der Formel (I) 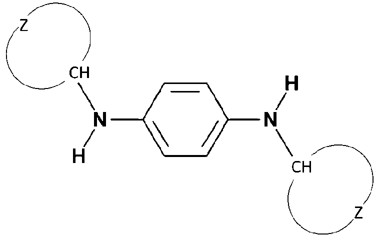 wobei eine Verbindung der Formel (II) 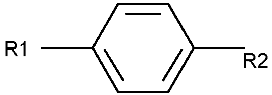
wobei R1 und R2 jeweils gleich oder verschieden sind und ausgewählt sind aus der Gruppe bestehend aus -NH₂ und -NO₂,
und wenigstens ein Keton der Formel (III) 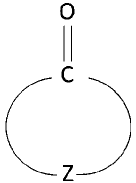
in Gegenwart eines Hydrierkatalysators und Wasserstoff umgesetzt werden,
wobei Z in den Formeln (I) und (III) gleich ist und
a) für eine Alkylenkette, bevorzugt eine lineare Alkylenkette, steht, die mit dem an Z angrenzenden Kohlenstoffatom einen cyclischen Ring bildet, und wobei die Alkylenkette von Z bevorzugt C₄-C₆-Alkylen, besonders bevorzugt C₅-Alkylen, ist,
oder
b) für zwei Alkylreste, bevorzugt lineare Alkylreste, steht, welche jeweils mit dem an Z angrenzenden Kohlenstoffatom verbunden sind und gemeinsam mit diesem Kohlenstoffatom eine Alkylkette, bevorzugt eine lineare Alkylkette, bilden, und wobei die zwei Alkylreste von Z gemeinsam die Summenformel CₙH₂ₙ₊₂ bilden, bevorzugt mit n=4-6, besonders bevorzugt mit n=5,
**dadurch gekennzeichnet, dass** die Umsetzung in Gegenwart eines Lösungsmittels der Formel (IVb) 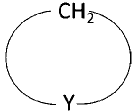
oder der Formel (IVc) 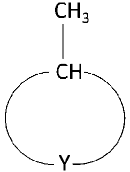
oder Mischungen davon,
erfolgt,
wobei Y in den Formeln (IVb) und (IVc) gleich oder verschieden, bevorzugt gleich, ist und
a) für eine Alkylenkette, bevorzugt eine lineare Alkylenkette, steht, die mit dem an Y angrenzenden Kohlenstoffatom einen cyclischen Ring bildet, und wobei die Alkylenkette von Y bevorzugt C₄-C₆-Alkylen, besonders bevorzugt C₅-Alkylen ist,
oder
b) für zwei Alkylreste, bevorzugt lineare Alkylreste, oder einen Alkylrest und ein Wasserstoffatom steht, welche jeweils mit dem an Y angrenzenden Kohlenstoffatom verbunden sind und gemeinsam mit diesem Kohlenstoffatom eine Alkylkette, bevorzugt eine lineare Alkylkette, bilden, und wobei die zwei Alkylreste oder der eine Alkylrest und das Wasserstoffatom gemeinsam die Summenformel CₙH₂ₙ₊₂ bilden, bevorzugt mit n=4-6, besonders bevorzugt mit n=5.

2. Verfahren gemäß Anspruch 1, **dadurch gekennzeichnet, dass** Z in den Formeln (I) und (III) für eine Alkylenkette, bevorzugt eine lineare Alkylenkette steht, die mit dem an Z angrenzenden Kohlenstoffatom eine cyclischen Ring bildet, und wobei die Alkylenkette von Z bevorzugt C₄-C₆-Alkylen, besonders bevorzugt C₅-Alkylen, ist.

3. Verfahren gemäß einem der Ansprüche 1 oder 2, **dadurch gekennzeichnet, dass** Y in den Formeln (IVb) und (IVc) gleich ist und für eine Alkylenkette, bevorzugt eine lineare Alkylenkette steht, die mit dem an Y angrenzenden Kohlenstoffatom eine cyclischen Ring bildet, und wobei die Alkylenkette von Y bevorzugt C₄-C₆-Alkylen, besonders bevorzugt C₅-Alkylen ist.

4. Verfahren gemäß einem de Ansprüche 1-3, **dadurch gekennzeichnet, dass** der Hydrierkatalysator 1 - 10 Gew.-% Platin, bevorzugt 1,5 - 5 Gew.-% Platin, besonders bevorzugt 2 - 4 Gew.-% Platin, ganz besonders bevorzugt 2,5 - 3,5 Gew.-% Platin, meist bevorzugt 3 Gew.-% Platin enthält.

5. Verfahren gemäß einem der Ansprüche 1-4, **dadurch gekennzeichnet, dass** das Lösungsmittel der Formel (IV) ausgewählt ist aus Cyclohexan und Methylcyclohexan.

6. Verfahren gemäß einem der Ansprüche 1-5, **dadurch gekennzeichnet, dass** die Umsetzung bei einem Wasserstoffdruck von 5 - 30 bar erfolgt, bevorzugt von 10 - 20 bar, besonders bevorzugt von 13 - 17bar.

7. Verfahren gemäß einem der Ansprüche 1-6, **dadurch gekennzeichnet, dass** die Umsetzung bei einer Temperatur von 100 - 170 °C erfolgt, bevorzugt bei 90 - 150 °C, besonders bevorzugt bei 95 - 140 °C, ganz besonders bevorzugt bei 125 - 135 °C.

8. Verfahren gemäß einem der Ansprüche 1-7, **dadurch gekennzeichnet, dass** die Umsetzungsdauer 50 min bis 400 min beträgt, bevorzugt 80 min bis 300 min, besonders bevorzugt 100 bis 270 min, ganz besonders bevorzugt 100 bis 200 min.

9. Verfahren gemäß einem der Ansprüche 1-8, **dadurch gekennzeichnet, dass** das Verfahren ein Verfahren zur Herstellung von *N,N'*-Dialkyl-*p*-phenylendiamin der Formel (I) in einer Reinheit von größer 95,0 Gew.-% ist, bevorzugt größer/gleich 96,0 Gew.-%, besonders bevorzugt größer als 97,0 Gew.-%, bevorzugt ermittelt mittels Gaschromatographie gemäß ASTM-D 4937.

10. Verfahren gemäß einem der Ansprüche 1-9, **dadurch gekennzeichnet, dass** das Verfahren ein Verfahren zur Herstellung von *N,N'*-Dialkyl-*p*-phenylendiamin der Formel (I) ist, welches die Verbindungen *N*-Alkyl-*p*-phenylendiamin sowie *N,N,N'*-Trialkyl-*p-*phenylendiamin in Summe kleiner als 3,0 Gew.-%, bevorzugt kleiner als 2,5 Gew.-%, besonders bevorzugt kleiner als 1,5 Gew.-%, meist bevorzugt kleiner als 1,0 Gew.-%, aufweist, bevorzugt ermittelt mittels Gaschromatographie gemäß ASTM-D 4937.

11. Verfahren gemäß einem der Ansprüche 1-10, **dadurch gekennzeichnet, dass** das wenigstens eine Keton der Formel (III) im molaren Verhältnis 1,2 : 1,0 bis 5,0 :1,0, bevorzugt 1,5 : 1,0 bis 3,0 : 1,0, besonders bevorzugt 1,8 : 1,0 bis 2,5 : 1,0, zu der Verbindung der Formel (II) eingesetzt wird.

12. Verfahren gemäß einem der Ansprüche 1-11, **dadurch gekennzeichnet, dass** das Verfahren ein Verfahren zur Herstellung von *N,N'*-Dicycloalkyl-*p*-phenylendiamin der Formel (I) ist, wobei Y und Z in den Formeln (I), (III) und (IVb) und (IVc) gleich sind und für eine lineare Alkylenkette stehen, welche bevorzugt C₅-Alkylen ist.

13. Verfahren gemäß einem der Ansprüche 9 oder 10, **dadurch gekennzeichnet, dass** die Bestimmung der Reinheit des *N,N'*-Dialkyl-*p*-phenylendiamins der Formel (I) gemäß Anspruch 9 sowie die Bestimmung der Anteile an *N*-Alkyl-*p*-phenylendiamin sowie *N,N,N'*-Trialkyl-*p*-phenylendiamin gemäß Anspruch 10, bevorzugt ermittelt mittels Gaschromatographie gemäß ASTM-D 4937, nach Entfernung des Hydrierkatalysators und des Lösungsmittels erfolgt.

## Claims

1. Process for preparing N,N'-dialkyl-p-phenylenediamine of the formula (I) 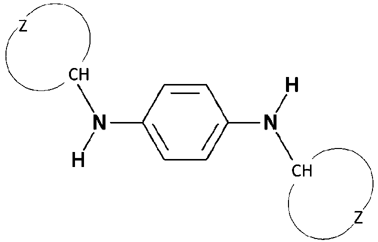 wherein a compound of the formula (II) 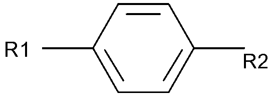
where R1 and R2 are each the same or different and are selected from the group consisting of -NH₂ and -NO₂,
and at least one ketone of the formula (III) 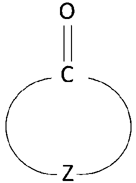
are converted in the presence of a hydrogenation catalyst and hydrogen,
where Z in the formulae (I) and (III) is the same and
a) is an alkylene chain, preferably a linear alkylene chain, which forms a cyclic ring with the carbon atom adjacent to Z, and where the alkylene chain of Z is preferably C₄-C₆-alkylene, more preferably C₅-alkylene,
or
b) represents two alkyl radicals, preferably linear alkyl radicals, that are each bonded to the carbon atom adjacent to Z and together with that carbon atom form an alkyl chain, preferably a linear alkyl chain, and where the two alkyl radicals of Z together form the empirical formula CₙH₂ₙ₊₂, preferably with n = 4-6, more preferably with n = 5,
**characterized in that** the reaction is effected in the presence of a solvent
of the formula (IVb) 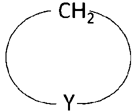
or of the formula (IVc) 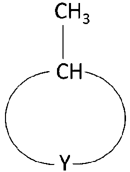
or mixtures thereof,
where Y in the formulae (IVb) and (IVc) is the same or different, preferably the same, and
a) is an alkylene chain, preferably a linear alkylene chain, which forms a cyclic ring with the carbon atom adjacent to Y, and where the alkylene chain of Y is preferably C₄-C₆-alkylene, more preferably C₅-alkylene,
or
b) represents two alkyl radicals, preferably linear alkyl radicals, or one alkyl radical and one hydrogen atom, that are each bonded to the carbon atom adjacent to Y and together with that carbon atom form an alkyl chain, preferably a linear alkyl chain, and where the two alkyl radicals or the one alkyl radical and the hydrogen atom together form the empirical formula CₙH₂ₙ₊₂, preferably with n = 4-6, more preferably with n = 5.

2. Process according to Claim 1, **characterized in that** Z in the formulae (I) and (III) is an alkylene chain, preferably a linear alkylene chain, which forms a cyclic ring with the carbon atom adjacent to Z, and where the alkylene chain of Z is preferably C₄-C₆-alkylene, more preferably C₅-alkylene.

3. Process according to either of Claims 1 and 2, **characterized in that** Y in the formulae (IVb) and (IVc) is the same and is an alkylene chain, preferably a linear alkylene chain, which forms a cyclic ring with the carbon atom adjacent to Y, and where the alkylene chain of Y is preferably C₄-C₆-alkylene, more preferably C₅-alkylene.

4. Process according to any of Claims 1-3, **characterized in that** the hydrogenation catalyst contains 1-10% by weight of platinum, preferably 1.5-5% by weight of platinum, more preferably 2-4% by weight of platinum, even more preferably 2.5-3.5% by weight of platinum, most preferably 3% by weight of platinum.

5. Process according to any of Claims 1-4, **characterized in that** the solvent of the formula (IV) is selected from cyclohexane and methylcyclohexane.

6. Process according to any of Claims 1-5, **characterized in that** the reaction is effected at a hydrogen pressure of 5-30 bar, preferably of 10-20 bar, more preferably of 13-17 bar.

7. Process according to any of Claims 1-6, **characterized in that** the reaction is effected at a temperature of 100-170°C, preferably at 90-150°C, more preferably at 95-140°C, even more preferably at 125-135°C.

8. Process according to any of Claims 1-7, **characterized in that** the reaction time is 50 min to 400 min, preferably 80 min to 300 min, more preferably 100 to 270 min, even more preferably 100 to 200 min.

9. Process according to any of Claims 1-8, **characterized in that** the process is a process for preparing N,N'-dialkyl-p-phenylenediamine of the formula (I) in a purity of greater than 95.0% by weight, preferably not less than 96.0% by weight, more preferably greater than 97.0% by weight, preferably ascertained by gas chromatography to ASTM-D 4937.

10. Process according to any of Claims 1-9, **characterized in that** the process is a process for preparing N,N'-dialkyl-p-phenylenediamine of the formula (I) that includes the compounds N-alkyl-p-phenylenediamine and N,N,N'-trialkyl-p-phenylenediamine in a sum total of less than 3.0% by weight, preferably less than 2.5% by weight, more preferably less than 1.5% by weight, most preferably less than 1.0% by weight, preferably ascertained by gas chromatography to ASTM-D 4937.

11. Process according to any of Claims 1-10, **characterized in that** the at least one ketone of the formula (III) is used in a molar ratio of 1.2 : 1.0 to 5.0 : 1.0, preferably 1.5 : 1.0 to 3.0 : 1.0, more preferably 1.8 : 1.0 to 2.5 : 1.0, to the compound of the formula (II).

12. Process according to any of Claims 1-11, **characterized in that** the process is a process for preparing N,N'-dicycloalkyl-p-phenylenediamine of the formula (I), where Y and Z in the formulae (I), (III) and (IVb) and (IVc) are the same and are a linear alkyl chain which is preferably C₅-alkylene.

13. Process according to either of Claims 9 and 10, **characterized in that** the purity of the N,N'-dialkyl-p-phenylenediamine of the formula (I) is determined according to Claim 9 and the proportions of N-alkyl-p-phenylenediamine and N,N,N'-trialkyl-p-phenylenediamine are determined according to Claim 10, preferably ascertained by gas chromatography to ASTM-D 4937, after removal of the hydrogenation catalyst and the solvent.

## Revendications

1. Procédé de préparation de N,N'-dialkyl-p-phénylènediamine de formule (I) 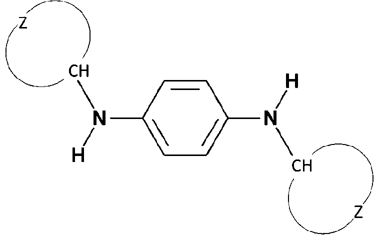 dans lequel on transforme un composé de formule (II) 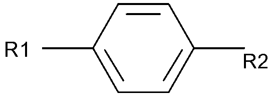
dans laquelle R1 et R2 sont à chaque fois identiques ou différents et choisis dans le groupe constitué par -NH₂ et -NO₂,
et au moins une cétone de formule (III) 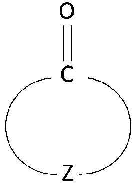
en présence d'un catalyseur d'hydrogénation et d'hydrogène,
Z dans les formules (I) et (III) étant identique et représentant
a) une chaîne alkylène, de préférence une chaîne alkylène linéaire, qui forme un noyau cyclique avec l'atome de carbone adjacent à Z et la chaîne alkylène de Z étant de préférence C₄-C₆-alkylène, de manière particulièrement préférée C₅-alkylène,
ou
b) deux radicaux alkyle, de préférence des radicaux alkyle linéaire, qui sont liés à chaque fois à l'atome de carbone adjacent à Z et qui forment, conjointement avec cet atome de carbone, une chaîne alkyle, de préférence une chaîne alkyle linéaire, et les deux radicaux alkyle de Z formant conjointement la formule brute CₙH₂ₙ₊₂, de préférence avec n = 4-6, de manière particulièrement préférée avec n = 5,
**caractérisé en ce que** la transformation a lieu en présence d'un solvant
de formule (IVb), 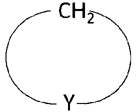
ou de formule (IVc) 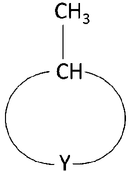
ou leurs mélanges,
,
Y dans les formules (IVb) et (IVc) étant identique ou différent, de préférence identique et représentant
a) une chaîne alkylène, de préférence une chaîne alkylène linéaire, qui forme un noyau cyclique avec l'atome de carbone adjacent à Y et la chaîne alkylène de Y étant de préférence C₄-C₆-alkylène, de manière particulièrement préférée C₅-alkylène,
ou
b) deux radicaux alkyle, de préférence des radicaux alkyle linéaire, ou un radical alkyle et un atome d'hydrogène, qui sont liés à chaque fois à l'atome de carbone adjacent à Y et qui forment, conjointement avec cet atome de carbone, une chaîne alkyle, de préférence une chaîne alkyle linéaire, et les deux radicaux alkyle ou ledit un radical alkyle et l'atome d'hydrogène formant conjointement la formule brute CₙH₂ₙ₊₂, de préférence avec n = 4-6, de manière particulièrement préférée avec n = 5.

2. Procédé selon la revendication 1, **caractérisé en ce que** Z dans les formules (I) et (III) représente une chaîne alkylène, de préférence une chaîne alkylène linéaire, qui forme un noyau cyclique avec l'atome de carbone adjacent à Z et la chaîne alkylène de Z étant de préférence C₄-C₆-alkylène, de manière particulièrement préférée C₅-alkylène.

3. Procédé selon l'une des revendications 1 ou 2, **caractérisé en ce que** Y dans les formules (IVb) et (IVc) est identique et représente une chaîne alkylène, de préférence une chaîne alkylène linéaire, qui forme un noyau cyclique avec l'atome de carbone adjacent à Y et la chaîne alkylène de Y étant de préférence C₄-C₆-alkylène, de manière particulièrement préférée C₅-alkylène.

4. Procédé selon l'une des revendications 1 à 3, **caractérisé en ce que** le catalyseur d'hydrogénation contient 1 à 10% en poids de platine, de préférence 1,5 à 5% en poids de platine, de manière particulièrement préférée 2 à 4% en poids de platine, de manière tout particulièrement préférée 2,5 à 3,5% en poids de platine, le plus préférablement 3% en poids de platine.

5. Procédé selon l'une des revendications 1 à 4, **caractérisé en ce que** le solvant de formule (IV) est choisi parmi le cyclohexane et le méthylcyclohexane.

6. Procédé selon l'une des revendications 1 à 5, **caractérisé en ce que** la transformation a lieu à une pression d'hydrogène de 5 à 30 bars, de préférence de 10 à 20 bars, de manière particulièrement préférée de 13 à 17 bars.

7. Procédé selon l'une des revendications 1 à 6, **caractérisé en ce que** la transformation a lieu à une température de 100 à 170°C, de préférence de 90 à 150°C, de manière particulièrement préférée de 95 à 140°C, de manière tout particulièrement préférée de 125 à 135°C.

8. Procédé selon l'une des revendications 1 à 7, **caractérisé en ce que** la durée de transformation est de 50 min à 400 min, de préférence de 80 min à 300 min, de manière particulièrement préférée de 100 à 270 min, de manière tout particulièrement préférée de 100 à 200 min.

9. Procédé selon l'une des revendications 1 à 8, **caractérisé en ce que** le procédé est un procédé de préparation de N,N'-dialkyl-p-phénylènediamine de formule (I) à une pureté supérieure à 95,0% en poids, de préférence supérieure ou égale à 96,0% en poids, de manière particulièrement préférée supérieure à 97,0% en poids, de préférence déterminée au moyen d'une chromatographie en phase gazeuse selon la norme ASTM-D 4937.

10. Procédé selon l'une des revendications 1 à 9, **caractérisé en ce que** le procédé est un procédé de préparation de N,N'-dialkyl-p-phénylènediamine de formule (I), qui présente les composés N-alkyl-p-phénylènediamine ainsi que N,N,N'-trialkyl-p-phénylènediamine au total en une quantité inférieure à 3,0% en poids, de préférence inférieure à 2,5% en poids, de manière particulièrement préférée inférieur à 1,5% en poids, le plus préférablement inférieure à 1,0% en poids, de préférence déterminée au moyen d'une chromatographie en phase gazeuse selon la norme ASTM-D 4937.

11. Procédé selon l'une des revendications 1 à 10, **caractérisé en ce que** ladite au moins une cétone de formule (III) est utilisée dans un rapport de 1,2:1,0 à 5,0:1,0, de préférence de 1,5:1,0 à 3,0:1,0, de manière particulièrement préférée de 1,8:1,0 à 2,5:1,0, au composé de formule (II).

12. Procédé selon l'une des revendications 1 à 11, **caractérisé en ce que** le procédé est un procédé de préparation de N,N'-dicycloalkyl-p-phénylènediamine de formule (I), Y et Z dans les formules (I), (III) et (IVb) et (IVc) étant identiques et représentant une chaîne alkyle linéaire qui est de préférence C₅-alkylène.

13. Procédé selon l'une des revendications 9 ou 10, **caractérisé en ce que** la détermination de la pureté de la N,N'-dialkyl-p-phénylènediamine de formule Formel (I) selon la revendication 9 ainsi que la détermination des proportions de N-alkyl-p-phénylènediamine ainsi que de N,N,N'-trialkyl-p-phénylènediamine selon la revendication 10, de préférence déterminées au moyen de chromatographie en phase gazeuse selon la norme ASTM-D 4937, ont lieu après l'élimination du catalyseur d'hydrogénation et du solvant.
